# EUROPÄISCHE PATENTSCHRIFT

(11) **EP 0 419 631 B1**
(45) Veröffentlichungstag und Bekanntmachung des Hinweises auf die Patenterteilung: **16.06.1993**
(21) Anmeldenummer: 90906045.1
(22) Anmeldetag: 17.04.1990
(51) Int. Cl.: C07C 37/20, C07C 39/17

(54) **VERFAHREN ZUR SYNTHESE VON 9,9-BIS(4-HYDROXYPHENYL)FLUOREN**
A PROCESS FOR SYNTHESISING 9,9-BIS(4-HYDROXYPHENYL) FLUORENE
PROCEDE POUR LA SYNTHESE DU 9,9-BIS(4-HYDROXYPHENYL)FLUORENE

(30) Priorität: 17.04.1989 AT 901/89
(43) Veröffentlichungstag der Anmeldung: 03.04.1991
(73) Patentinhaber: ISONOVA TECHNISCHE INNOVATIONEN GES.M.B.H., A-2355 Wiener Neudorf (AT)
(72) Erfinder: FIALLA, Peter, A-2344 Maria Enzersdorf (AT)
(74) Vertreter: Stampfer, Heinz
(86) Internationale Anmeldenummer: AT9000031
(87) Internationale Veröffentlichungsnummer: WO9012776

(56) Entgegenhaltungen:
- EP-A- 0 065 060
- EP-A- 0 180 133
- DE-A- 2 508 710

## Beschreibung

Die Erfindung betrifft ein Verfahren zum Herstellen von 9,9-Bis(4-hydroxyphenyl)fluoren durch Umsetzung von Fluorenon mit Phenol in Überschuß in einer Kondensationsreaktion unter Anwendung von Chlorwasserstoffgas als Kondensationsmittel sowie von Aluminiumtrichlorid als Katalysator.

### Stand der Technik

Aus der EP-A-0065060 ist ein Verfahren dieser Art bekannt, bei dem der feste Katalysator wie z.B. AlCl₃ dem Reaktionsgemisch beigefügt und das Chlorwasserstoffgas während der Reaktion in das Reaktionsgemisch eingeleitet wird. Dabei ist wichtig, daß die Temperatur im Reaktionsgefäß einen Wert von 60 - 80 ° C nicht übersteigt. Mit diesem Verfahren konnte die Bildung von Isomeren des 9,9-Bis(4-hydroxyphenyl)fluoren sowie von höher Kondensierten Nebenprodukten weitgehend hintangehalten werden. Bei Syntheseansätzen in großen Kesseln zeigt das Verfahren aber einige verfahrenstechnische Nachteile. Einer dieser Nachteile besteht zunächst darin, daß der feste pulverförmige Katalysator (AlCl₃) im Reaktionsgemisch nicht in Suspension gehalten werden kann, sondern sich bald am Boden des Reaktionsgefäßes absetzt, und die Gefahr besteht, daß er die Bodenauslaßventile des Kessels verstopft. Ferner ist die Einleitung von Chlorwasserstoffgas über den Reaktionszeitraum, der mehrere Stunden beträgt, nicht einfach zu realisieren. Schließlich kann bei diesem bekannten Verfahren, speziell bei großen Massen und zunehmender Viskosität, die Exothermie nur mehr mangelhaft über die Wandkühlung beherrscht werden.

### Darstellung der Erfindung

Der Erfindung liegt nun die Aufgabe zugrunde, ein Verfahren der eingangs genannten Art anzugeben, das diese Nachteile nicht aufweist.

Die der Erfindung zugrunde liegende Aufgabe wird in dem erfindungsgemäßen Verfahren dadurch gelöst, daß zur Herstellung eines flüssigen Katalysators Aluminiumchlorid in einem wasserfreien organischen Lösungsmittel oder Lösungsmittelgemisch, das gegenüber der Kondensationsreaktion zumindest nahezu inert ist, wobei das Lösungsmittel bzw. zumindest eine Komponente des Lösungsmittelsgemisches die Eigenschaft eines Elektronendonors hat, unter Einleitung von Chlorwasserstoffgas gelöst wird und dieser flüssige Katalysator dem Reaktionsgemisch unter Rühren zugetropft wird, wobei insbesondere durch Steuerung der Zugaberate des Katalysators die Temperatur des Reaktionsgemisches während dieses Zutropfens unter 60° C gehalten wird.

Nach einer vorteilhaften Ausgestaltung des erfindungsgemäßen Verfahrens ist das Lösungsmittel bzw. eine Komponente des Lösungsmittelgemisches Benzol oder ein substituiertes Benzol, vorteilhaft Toluol.

Gemäß einer anderen vorteilhaften Ausgestaltung der Erfindung ist das Lösungsmittel bzw. eine Komponente des Lösungsmittelgemisches ein Ather. Dabei kann dieser Ather vorteilhaft Methyl-tert.-Butyläther sein.

### Ein Weg zur Ausführung der Erfindung

a) Eingesetzte Stoffe:
   - 9010 g (50 Mol) Fluorenon einer Reinheit von 99,0 % mit einem Schmelzpunkt von 80-82° C und einer Dichte von 1,13 g/cm³
   - 18820 g (200 Mol) Phenol (Wassergehalt 0,2 %)
   - 1300 g (9,75 Mol) sublimiertes Aluminiumtrichlorid
   - Chlorwasserstoffgas
   - wasserfreies Toluol
b) Herstellung des flüssigen Katalysators:
   In einem mit einem Rührer versehenen Kolben werden drei Liter wasserfreies Toluol und die angegebene Menge Aluminiumchlorid vorgelegt. Danach wird bei Raumtemperatur Chlorwasserstoffgas unter ständigem Rühren in den Kolben eingeleitet, bis sich das ganze Aluminiumtrichlorid gelöst hat und ein zweiphasiges, flüssiges System entstanden ist. Die überstehende Phase besteht dabei hauptsächlich aus Toluol und darin gelöstem Chlorwasserstoffgas.
c) Durchführung der Synthese :
   Das feste Flurenon wird in einem 100 l-Reaktionsgefäß vorgelegt und mit auf etwa 60 °C erwärmten, also flüssigem Phenol unter Rühren versetzt. Das Reaktionsgemisch wird auf 40 bis 50 ° C erwärmt. Danach wird unter weiterem Rühren der flüssige Katalysator langsam zugetropft, so daß die Temperatur auf ca. 50 °C gehalten wird. Das Zutropfen des Katalysators nimmt dabei etwa 1,5 h in Anspruch.
   Nach Beendigung der Katalysatorzugabe ist ein Ansteigen der Viskosität des Reaktionsgemisches, aus dem das Rohprodukt schon zum Teil ausfällt, erkannbar. Etwa 4 bis 5 Stunden nach Reaktionsbeginn werden 20 l heißem Wassers zugegeben und danach das überschüssige Phenol mittels Wasserdampfdestillation entfernt.
   Nach Phasentrennung wird das überstehende Wasser dekantiert und das Rohprodukt mit etwa 30 l heißem Wasser gewaschen, es wird erneut absetzen gelassen und dekantiert. Danach wird das so behandelte Rohprodukt mit 30 l Aceton unter Rückflußkochen gelöst. Die acetonische Lösung wird gekühlt und es kristallisiert ein Acetonaddukt des 9,9-Bis(4-hydroxyphenyl)fluorens aus. Dieses kristalline Acetonaddukt wird im Vakuumofen bei 100 °C und einem Druck von 0.1 bar getrocknet. Die Ausbeute an Rohprodukt beträgt bei dem beschriebenen Verfahren 85 - 90 % der Theorie.
   Dieses Rohprodukt wird aus 1,2-Dichloräthan umkristallisiert. Dabei wird es im Dichloräthan unter Rückfluß gelöst und anschliessend über Aktivkohle filtriert. Das unter Kühlen auskristallisierte Reinprodukt mit einer Reinheit von größer als 99,8 % fällt in einer Ausbeute von etwa 80 % der Theorie an.
   Der mittels DSC als onset-Temperatur gemessene Schmelzpunkt des reinen 9,9-Bis(4-hydroxyphenyl)fluorens beträgt 225,5 °C.

### Gewerbliche Verwendbarkeit

Das gemäß dem erfindungsgemäßen Verfahren hergestellte 9,9-Bis(4-hydroxyphenyl)fluoren kann vorteilhaft, analog wie in der EP-A-0065060 näher erläutert, als Monomer zur Herstellung hochtemperaturbeständiger aromatischer Polyester eingesetzt werden.

## Patentansprüche

1. Verfahren zum Herstellen von 9,9-Bis(4-hydroxyphenyl)fluoren durch Umsetzung von Fluorenon mit Phenol in Überschuß in einer Kondensationsreaktion unter Anwendung von Chlorwasserstoffgas als Kondensationsmittel sowie von Aluminiumtrichlorid als Katalysator, dadurch gekennzeichnet, daß zur Herstellung eines flüssigen Katalysators das Aluminiumtrichlorid in einem wasserfreien organischen Lösungsmittel oder Lösungsmittelgemisch, das gegenüber der Kondensationsreaktion zumindest nahezu inert ist, wobei das Lösungsmittel bzw. zumindest eine Komponente des Lösungsmittelgemisches die Eigenschaft eines Eleketronendonors hat, unter Einleitung von Chlorwasserstoffgas gelöst wird und daß dieser flüssige Katalysator dem Reaktionsgemisch unter Rühren zugetropft wird, wobei insbesondere durch Steuerung der Zugaberate des Katalysators die Temperatur des Reaktionsgemisches während dieses Zutropfens unter 60° C gehalten wird.

2. Verfahren nach Anspruch 1, dadurch gekennzeichnet, daß das Lösungsmittel bzw. eine Komponente des Lösungsmittelgemisches Benzol oder ein substituiertes Benzol, vorzugsweise Toluol, ist.

3. Verfahren nach Anspruch 1, dadurch gekennzeichnet, daß das Lösungsmittel bzw. eine Komponente des Lösungsmittelgemisches ein Äther, vorgzusweise Methyl-tert.-Butyläther, ist.

## Claims

1. A method for the preparation of 9,9-bis(4-hydroxyphenyl)fluorene by reacting fluorenone with phenol in an excess in a condensation reaction using hydrogen chloride gas as a condensation agent and aluminium trichloride as a catalyst, characterised in that in order to produce a liquid catalyst the aluminium trichloride is dissolved, with the introduction of hydrogen chloride gas, in an anhydrous organic solvent or solvent mixture which is at least virtually inert to the condensation reaction, the solvent or at least one constituent of the solvent mixture having the property of an electron donor, and that this liquid catalyst is added drop-wise to the reaction mixture with stirring, the temperature of the reaction mixture during this drop-wise addition being kept below 60°C, in particular by controlling the rate of addition of the catalyst.

2. A method according to Claim 1, characterised in that the solvent or a constituent of the solvent mixture is benzene or a substituted benzene, preferably toluene.

3. A method according to Claim 1, characterised in that the solvent or a constituent of the solvent mixture is an ether, preferably methyl tert. butyl ether.

## Revendications

1. Procédé de préparation de 9,9-bis(4-hydroxyphényl)fluorène par conversion de fluorène avec du phénol en excès, selon une réaction de condensation dans laquelle du HCl gazeux comme agent de condensation et du trichlorure d'aluminium agit comme catalyseurs, caractérisé en ce que, pour l'obtention d'un catalyseur fluide, le trichlorure d'aluminium est dissous, sous l'action d'HCl gazeux dans un solvant ou mélange de solvant(s) organique(s) anhydre(s), au moins sensiblement inerte(s) dans les conditions de la réaction de condensation, ledit solvant ou au moins un composant dudit mélange de solvants ayant un caractère électrodonneur, et en ce que ce catalyseur fluide est ajouté au mélange réactionnel, goutte à goutte et sous agitation la température du milieu réactionnel pendant cet ajout goutte à goutte étant maintenue à 60°C, en particulier par contrôle de la vitesse d'addition dudit catalyseur.

2. Procédé selon la revendication 1, caractérisé en ce que ledit solvant ou un composant dudit mélange de solvant(s) est du benzène ou un benzène substitué, de préférence du toluène.

3. Procédé selon la revendication 1, caractérisé en ce que, ledit solvant ou un composant dudit mélange de solvant(s) est un éther, de préférence le méthyltertiobutyléther.
